Europäisches Patentamt

European Patent Office

Office européen des brevets

(11) Veröffentlichungsnummer: **0 443 138 A1**

(12) **EUROPÄISCHE PATENTANMELDUNG**

(21) Anmeldenummer: 90124222.2

(22) Anmeldetag: **14.12.90**

(51) Int. Cl.5: **C08G 18/79,** C08G 18/80,
C07D 229/00, C08G 18/08,
C09D 175/04

(30) Priorität: **23.01.90 DE 4001783**

(43) Veröffentlichungstag der Anmeldung:
**28.08.91 Patentblatt 91/35**

(84) Benannte Vertragsstaaten:
**AT BE CH DE ES FR GB IT LI NL SE**

(71) Anmelder: **BAYER AG**

**W-5090 Leverkusen 1 Bayerwerk(DE)**

(72) Erfinder: **Mosbach, Jürgen, Dr.**
**Katterbachstrasse 108**
**W-5060 Bergisch, Gladbach(DE)**
Erfinder: **Laas, Hans-Josef, Dr.**
**Merheimerstrasse 249**
**W-5000 Köln 60(DE)**
Erfinder: **Kubitza, Werner**
**Eduard-Spranger-Strasse 22**
**W-5090 Leverkusen 3(DE)**

(54) **Polyisocyanatgemische, Verfahren zu ihrer Herstellung und ihre Verwendung als Bindemittel für Überzugsmittel oder als Reaktionspartner für gegenüber Isocyanatgruppen oder Carboxylgruppen reaktionsfähige Verbindungen.**

(57) Polyisocyanatgemische, gekennzeichnet durch
a) eine mittlere NCO-Funktionalität von 1,5 bis 4,0,
b) einen Gehalt an (cyclo)aliphatisch gebundenen Isocyanatgruppen von 2 bis 40 Gew.-%,
c) einen Gehalt an chemisch fixierten Carboxylgruppen von 0,01 bis 15 Gew.-% und
d) einen Gehalt an (cyclo)aliphatisch gebundenen Uretdiongruppen von 1 bis 23 Gew.-%,
Verfahren zu ihrer Herstellung durch Umsetzung von (cyclo)aliphatisch gebundene Uretdiongruppen aufweisenden Diisocyanaten, gegebenenfalls in Abmischung mit weiteren Polyisocyanaten mit einer Hydroxycarbonsäure-Komponente und gegebenenfalls weiteren Reaktionspartnern mit gegenüber Isocyanatgruppen reaktionsfähigen Hydroxylgruppen oder durch Modifizierung von Uretdiongruppen-freien Polyisocyanaten mit Hydroxycarbonsäuren und anschließender Dimerisierung eines Teils der danach noch vorliegenden Isocyanatgruppen und die Verwendung der Polyisocyanatgemische als Bindemittel für Überzugsmittel oder als Reaktionspartner für Verbindungen mit gegenüber Isocyanatgruppen und/oder Carboxylgruppen reaktionsfähigen Gruppen bei der Herstellung von hochmolekularen Kunststoffen.

EP 0 443 138 A1

# POLYISOCYANATGEMISCHE, VERFAHREN ZU IHRER HERSTELLUNG UND IHRE VERWENDUNG ALS BINDEMITTEL FÜR ÜBERZUGSMITTEL ODER ALS REAKTIONSPARTNER FÜR GEGENÜBER ISOCYANAT-GRUPPEN ODER CARBOXYLGRUPPEN REAKTIONSFÄHIGE VERBINDUNGEN

Die Erfindung betrifft neue Polyisocyanatgemische, die sowohl Carboxylgruppen als auch (cyclo)-aliphatisch gebundene Uretdiongruppen aufweisen, und die daher vielseitig verwendbare Vorprodukte für die Herstellung von Kunststoffen darstellen, Verfahren zur Herstellung dieser Polyisocyanatgemische durch Modifizierung von organischen Polyisocyanaten mit (cyclo)aliphatisch gebundenen Isocyanatgruppen mit aliphatischen Hydroxycarbonsäuren unter Mitverwendung von Uretdiongruppen aufweisenden Polyisocyanaten oder durch Modifizierung von organischen Polyisocyanaten mit Hydroxycarbonsäuren und anschließende Dimerisierung eines Teils der verbleibenden Isocyanatgruppen und die Verwendung der Polyisocyanatgemische als Bindemittel für Überzugsmittel oder als Reaktionspartner für gegenüber Isocyanat- oder Carboxylgruppen reaktionsfähige Verbindungen bei der Herstellung von hochmolekularen Kunststoffen.

Uretdiongruppen aufweisende Polyisocyanate stellen interessante, hitze-aktivierbare, abspalterfreie Vernetzungsmittel für Kunststoffvorläufer mit gegenüber Isocyanatgruppen reaktionsfähigen Gruppen dar, da sie sowohl bei Raumtemperatur (freie NCO-Gruppen) als auch bei höheren Temperaturen (blockierte NCO-Gruppen) mit diesen im Sinne einer Additionsreaktion zu reagieren vermögen. So entstehen beispielsweise aus Hydroxylgruppen und Uretdiongruppen bei erhöhten Temperaturen Allophanatgruppen. Von technischer Bedeutung sind hierbei u.a. Verbindungen mit cycloaliphatisch gebundenen Uretdiongruppen (DE-AS 3 030 513) bzw, mit aliphatisch gebundenen Uretdiongruppen (DE-OS 3 437 635).

Auch Isocyanatgruppen und freie Carboxylgruppen aufweisende Verbindungen sind als Kunststoffvorläufer bekannt geworden. So können beispielsweise bei der Synthese wäßriger Polyurethandispersionen durch Verwendung sterisch gehinderter Hydroxyalkancarbonsäuren - wie z.B. Dimethylolpropionsäure - eingebaute Carboxylgruppen tragende NCO-Polyurethanprepolymere als Zwischenstufen hergestellt werden (US-P 3 412 054).

Überraschenderweise wurde jetzt gefunden, daß die Kombination dieser beiden Prinzipien, d.h, der gleichzeitige Einbau von (cyclo)aliphatisch gebundenen Uretdiongruppen und Carboxylgruppen zu entsprechend modifizierten Polyisocyanatgemischen führt, die eine Reihe bemerkenswerter Eigenschaften in sich vereinen. Diese nachstehend näher beschriebenen erfindungsgemäßen Polyisocyanatgemische sind bei Raumtemperatur trotz des gleichzeitigen Vorliegens von Isocyanat- und Carboxylgruppen während mindestens 3 Monaten lagerstabil, sie sind in mit tert.- Aminen neutralisierter Form in Wasser löslich bzw. dispergierbar, sie sind in der Hitze selbstvernetzend, sie weisen die interessante Eigenschaft auf, auch ohne Zusatz weiterer Hilfs- und Zusatzmittel filmbildend zu sein, und sie sind selbstverständlich wegen des gleichzeitigen Vorliegens von drei unterschiedlichen Reaktivgruppen den unterschiedlichsten Reaktionen mit entsprechenden Reaktionspartnern zugänglich. Insbesondere eignen sie sich in Kombination mit Verbindungen mit gegenüber Isocyanatgruppen reaktionsfähigen Gruppen zur Herstellung von interessanten hochmolekularen Kunststoffen.

Gegenstand der Erfindung sind Polyisocyanatgemische, gekennzeichnet durch

a) eine mittlere NCO-Funktionalität von 1,5 bis 4,0,

b) einen Gehalt an (cyclo)aliphatisch gebundenen Isocyanatgruppen (NCO, Molekulargewicht = 42) von 2 bis 40 Gew.-%,

c) einen Gehalt an chemisch fixierten Carboxylgruppen (COOH, Molekulargewicht = 45) von 0,01 bis 15 Gew.-% und

d) einen Gehalt an (cyclo)aliphatisch gebundenen Uretdiongruppen ($C_2N_2O_2$, Molekulargewicht = 84) von 1 bis 23 Gew.-%.

Gegenstand der Erfindung ist auch ein 1. Verfahren zur Herstellung dieser Polyisocyanate durch Umsetzung einer Polyisocyanatkomponente A), bestehend aus mindestens einem Polyisocyanat mit (cyclo)-aliphatisch gebundenen Isocyanatgruppen des Molekulargewichtsbereichs 168 bis 1000 mit einer Hydroxycarbonsäure-Komponente B), bestehend aus mindestens einer aliphatischen Hydroxycarbonsäure des Molekulargewichtsbereichs 76 bis 200, gegebenenfalls unter Mitverwendung von weiteren Reaktionspartnern mit gegenüber Isocyanatgruppen reaktionsfähigen, aliphatisch gebundenen Hydroxylgruppen unter Einhaltung eines Äquivalentverhältnisses von Isocyanatgruppen zu Hydroxylgruppen von 1,05:1 bis 80:1 und gegebenenfalls anschließende destillative Entfernung von leicht flüchtigen, nicht umgesetzten Ausgangspolyisocyanaten und/oder gegebenenfalls anschließende Abmischung des resultierenden Umsetzungsprodukts mit weiteren organischen Polyisocyanaten C), dadurch gekennzeichnet, daß man als Polyisocyanatkomponente A) oder als Teil der Polyisocyanatkomponente A) und/oder als Polyisocyanate C) oder als Teil der Polyisocyanate C) (cyclo)aliphatisch gebundene Uretdiongruppen aufweisende Diisocyanate mit (cyclo)aliphatisch gebundenen Isocyanatgruppen verwendet, wobei im übrigen Art und Mengenverhältnisse der Reaktionspartner so gewählt werden, daß die resultierenden Polyisocyanatgemische den oben unter a) bis d) genannten Bedingungen entsprechen.

Gegenstand der Erfindung ist ferner ein 2. Verfahren zur Herstellung dieser Polyisocyanate, welches dadurch gekennzeichnet ist, daß man eine Polyisocyanatkomponente A), bestehend aus mindestens einem, im wesentlichen Uretdiongruppen-freien Polyisocyanat mit (cyclo)aliphatisch gebundenen Isocyanatgruppen des Molekulargewichtsbereichs 168 bis 1000 mit einer Hydroxycarbonsäure-Komponente B), bestehend aus mindestens einer aliphatisch gebundenen Hydroxycarbonsäure des Molekulargewichtsbereichs 76 bis 200, gegebenenfalls unter Mitverwendung von weiteren Reaktionspartnern mit gegenüber Isocyanatgruppen reaktionsfähigen, aliphatisch gebundenen Hydroxylgruppen unter Einhaltung eines Äquivalentverhältnisses von Isocyanatgruppen zu Hydroxylgruppen von 1,05:1 bis 400:1 umsetzt, die verbleibenden Isocyanatgruppen des Umsetzungsprodukts zu 5 bis 60 % in Gegenwart eines die Dimerisierung von Isocyanatgruppen beschleunigenden Katalysators in Uretdiongruppen und gegebenenfalls Isocyanuratgruppen überführt, das dann vorliegende Reaktionsgemisch gegebenenfalls von überschüssigem Ausgangspolyisocyanate A) befreit und/oder mit weiteren organischen Polyisocyanaten C) abmischt, wobei Art und Mengenverhältnisse der Reaktionspartner und die Menge des gegebenenfalls am Ende der Umsetzung entfernten Ausgangspolyisocyanats A) und/oder des dem Reaktionsgemisch zugesetzten Polyisocyanat C) so bemessen werden, daß die resultierenden Polyisocyanatgemische den oben unter a) bis d) genannten Bedingungen entsprechen.

Gegenstand der Erfindung ist auch die Verwendung der Polyisocyanatgemische gegebenenfalls in zumindest teilweise mit tertiären Aminen neutralisierter Form als Bindemittel für unter dem Einfluß von Feuchtigkeit und/oder Hitze vernetzbaren Überzugsmitteln.

Gegenstand der Erfindung ist schließlich auch die Verwendung der Polyisocyanatgemische gegebenenfalls in zumindest teilweise mit tertiären Aminen neutralisierter Form, als Reaktionspartner für Verbindungen mit gegenüber Isocyanatgruppen und/oder Carboxylgruppen reaktionsfähigen Gruppen bei der Herstellung von hochmolekularen Kunststoffen.

Die beim 1. erfindungsgemäßen Verfahren einzusetzende Polyisocyanatkomponente A) besteht aus mindestens einem Polyisocyanat des Molekulargewichtsbereichs 168 bis 1000 mit (cyclo)aliphatisch gebundenen Isocyanatgruppen und einer NCO-Funktionalität von 2 bis 4, vorzugsweise 2 bis 3. Geeignet sind beispielsweise einfache organische Diisocyanate wie z.B. 1,6-Diisocyanatohexan (HDI), 1-Isocyanato-3,5,5-trimethyl-5-isocyanatomethyl-cyclohexan (IPDI), 4,4'-Diisocyanato-dicyclohexylmethan oder auch Modifizierungsprodukte derartiger einfacher Diisocyanate wie z.B. Polyisocyanate mit Oxadiazintrionstruktur wie sie aus 2 Mol eines derartigen Diisocyanats, insbesondere 2 Mol HDI mit 1 Mol Kohlendioxid entstehen, Biuretgruppen aufweisende Polyisocyanate wie insbesondere N,N',N''-Tris-(isocyanatohexyl)-biuret oder seine Gemische mit seinen höheren Homologen oder Isocyanuratgruppen aufweisende Polyisocyanate auf Basis der beispielhaft genannten einfachen Diisocyanate, insbesondere auf Basis von HDI und/oder IPDI oder Uretdiongruppen aufweisende Diisocyanate wie beispielsweise dimeres HDI oder dimeres IPDI oder beliebige Gemische derartiger Polyisocyanate, soweit sie den obengemachten Bedingungen entsprechen.

Beim 2. erfindungsgemäßen Verfahren werden ausschließlich Ausgangspolyisocyanate A) der genannten Art eingesetzt, die keine Uretdiongruppen aufweisen, ansonsten jedoch den obengemachten Ausführungen entsprechen.

Die bei beiden erfindungsgemäßen Verfahren einzusetzende Hydroxycarbonsäurekomponente B) besteht aus mindestens einer aliphatischen Hydroxycarbonsäure des Molekulargewichtsbereichs 76 bis 200 wie beispielsweise 2-Hydroxyessigsäure, 4-Hydroxybuttersäure, 2,2-Bis-(hydroxymethyl)-propionsäure (DMPS). Besonders bevorzugt wird DMPS als Hydroxycarbonsäurekomponente B) verwendet.

Bei der Durchführung beider erfindungsgemäßen Verfahren können als weitere Reaktionspartner gegebenenfalls weitere Aufbaukomponenten mit aliphatisch gebundenen Hydroxylgruppen eingesetzt werden. Bei diesen Verbindungen handelt es sich vorzugsweise um nichtionisch hydrophile Aufbaukomponenten, die zur Hydrophilie der erfindungsgemäßen Polyisocyanatgemische beitragen. In diesem Zusammenhang sind beispielsweise hydrophile Seitenketten aufweisende Diole der in US-PS 4 190 566 genannten Art oder hydrophile einwertige Polyetheralkohole der in US-PS 4 237 264 genannten Art beispielhaft zu erwähnen. Weitere gegebenenfalls mitzuverwendende Verbindungen mit aliphatisch gebundenen Hydroxylgruppen sind beispielsweise niedermolekulare Kettenverlängerungsmittel oder Vernetzer wie Ethylenglykol, Propylenglykol, Trimethylolpropan oder Gemische derartiger niedermolekularer Polyhydroxylverbindungen, die im allgemeinen ein maximales Molekulargewicht von 200 aufweisen. Auch einbaufähige Emulgatoren der an sich bekannten Art wie beispielsweise ethoxylierte Alkylphenole, insbesondere ethoxylierte Nonylphenole, oder Ether- bzw. Estergruppen aufweisende einbaufähige Emulgatoren wie beispielsweise Polyoxyethylenlaurylether oder Polyoxyethylen-ester höherer Fettsäuren wie beispielsweise Polyoxyethylenlaurat, -oleat oder -stearat können als weitere Aufbaukomponente mit einer aliphatischen Hydroxylgruppe mitverwendet werden. Diese einbaufähigen Emulgatoren weisen im allgemeinen pro Molekül 8 bis 50 Oxyethyleneinheiten auf.

Die beispielhaft genannten Verbindungen mit alkoholischen Hydroxylgruppen können bei der Durchführung beider erfindungsgemäßen Verfahren in Mengen von bis zu 10 Gew.-%, bezogen auf das Gewicht der Komponenten A) und B) mitverwendet werden,

Bei der Durchführung des 1. erfindungsgemäßen Verfahrens erfolgt die Umsetzung der beispielhaft genannten Ausgangsmaterialien im allgemeinen unter Einhaltung eines Äquivalentverhältnisses von Isocyanatgruppen zu Hydroxylgruppen von 1,05:1 bis 80:1, vorzugsweise 1,2:1 bis 50:1 bei 20 bis 100, vorzugsweise 25 bis 90°C. Die Umsetzung kann in Substanz oder auch in Gegenwart von geeigneten, gegenüber Isocyanatgruppen inerten Lösungsmitteln durchgeführt werden.

Im Anschluß an die erfindungsgemäße Umsetzung können die Umsetzungsprodukte beider erfindungsgemäßen Verfahren gegebenenfalls mit weiteren organischen Polyisocyanaten C) abgemischt werden. Geeignete weitere Polyisocyanate sind insbesondere Uretdiongruppen aufweisende Polyisocyanate mit aliphatisch gebundenen Uretdiongruppen der bereits oben in Zusammenhang mit der Komponente A) beispielhaft genannten Art. Weitere denkbare Abmischkomponenten sind beispielsweise aromatische Uretdiondiisocyanate wie beispielsweise dimerisiertes oder trimerisiertes 2,4-Diisocyanatotoluol oder sonstige Polyisocyanate der aus der Polyurethanchemie an sich bekannten Art, beispielsweise solchen, wie sie bereits oben als Ausgangskomponente A) für das erfindungsgemäße Verfahren beispielhaft erwähnt werden.

Bei der Durchführung des 1. erfindungsgemäßen Verfahrens werden die Art und Mengenverhältnisse der Reaktionspartner, sowie die Art und Menge der gegebenenfalls noch nachträglich zugemischten Polyisocyanate C) im Rahmen der gemachten Offenbarung so bemessen, daß die resultierenden erfindungsgemäßen Polyisocyanatgemische den obengemachten Angaben a) bis d) entsprechen. Die besonders bevorzugten erfindungsgemäßen Polyisocyanatgemische weisen eine mittlere NCO-Funktionalität von 1,5 bis 4, insbesondere 1,5 bis 3, einen NCO-Gehalt von 2 bis 40, insbesondere 5 bis 20 Gew.-%, einen Carboxylgruppengehalt von 0,01 bis 15, insbesondere 0,5 bis 5 Gew.%, und einen Gehalt an aliphatisch gebundenen Uretdiongruppen von 1 bis 23, insbesondere 5 bis 20 Gew.-%, auf.

Bei der Durchführung des 2. erfindungsgemäßen Verfahrens werden, wie bereits ausgeführt, als Ausgangspolyisocyanate A) ausschließlich solche eingesetzt, die im wesentlichen frei von Uretdiongruppen sind, Diese Ausgangspolyisocyanate werden dann mit Hydroxycarbonsäuren der oben beispielhaft genannten Art gegebenenfalls unter Mitverwendung von weiteren Verbindungen mit aliphatisch gebundenen Hydroxylgruppen der ebenfalls oben beispielhaft genannten Art unter Einhaltung eines NCO/OH-Äquivalentverhältnisses von 1,05:1 bis 400:1 zur Reaktion gebracht. Diese Umsetzung erfolgt vorzugsweise in Substanz bei 25 bis 90°C.

In einem 2. Reaktionsschritt werden die Isocyanatgruppen des hierbei anfallenden Reaktionsgemischs dann zu 5 bis 60 %, vorzugsweise zu 10 bis 50 % in Gegenwart eines die Dimerisierung von Isocyanatgruppen beschleunigenden Katalysators in Uretdiongruppen und gegebenenfalls Isocyanuratgruppen überführt. Dies bedeutet, daß bei dieser Oligomerisierungsreaktion bis zu 80 %, vorzugsweise bis zu 50 % der während der Reaktion umgesetzten Isocyanatgruppen in Isocyanuratgruppen und zum Rest in Uretdiongruppen überführt werden, wobei auch höhere und/oder gemischte Homologe der entsprechenden Oligomerisierungsprodukte entstehen können. Diese Oligomerisierungsreaktion erfolgt im allgemeinen im Temperaturbereich von 25 bis 100, vorzugsweise 25 bis 90°C, wobei durch geeignete Wahl des Katalysators und insbesondere der Reaktionstemperatur das Verhältnis von Uretdiongruppen zu Isocyanuratgruppen im Oligomerisierungsprodukt gesteuert werden kann.

Als Katalysatoren kommen die hierfür an sich bekannten tertiären Phosphine in Betracht (DE-OS 1 670 720, 1 934 763, 3 030 513 oder US-PS 4 614 785). Besonders gut geeignet sind Tri-n-butyl- oder Tri-n-octylphosphin.

Die Abstoppung der Oligomerisierungsreaktion erfolgt in an sich bekannter Weise mittels geeigneter Katalysatorengifte wie z.B. p-Toluolsulfonsäuremethylester oder Schwefel oder aber auch durch destillative Entfernung des Katalysators aus dem Reaktionsgemisch.

Im Anschluß an die Oligomerisierungsreaktion erfolgt gegebenenfalls eine Entfernung des noch im Überschuß vorliegenden Ausgangspolyisocyanats A), beispielsweise durch Dünnschichtdestillation, sowie gegebenenfalls eine hieran anschließende Abmischung mit weiteren organischen Polyisocyanaten C).

Auch beim 2. erfindungsgemäßen Verfahren werden Art und Mengenverhältnisse der Reaktionspartner, sowie die Menge des gegebenenfalls am Anschluß an die Umsetzung entfernten Ausgangspolyisocyanats A), sowie Art und Menge des gegebenenfalls zugemischten Polyisocyanats C) so bemessen, daß die resultierenden erfindungsgemäßen Polyisocyanatgemische den obengemachten Angaben a) bis d) entsprechen. Die obengemachten Ausführungen bezüglich der Kennzahlen der besonders bevorzugten erfindungsgemäßen Polyisocyanatgemische gelten auch für die Verfahrensprodukte des 2. erfindungsgemäßen Verfahrens.

Aufgrund des gleichzeitigen Vorliegens von drei unterschiedlichen Reaktionszentren stellen die erfindungsgemäßen Polyisocyanatgemische wertvolle, vielseitig anwendbare Zwischenprodukte bzw. Vernetzer bei der Herstellung von hochmolekularen Kunststoffen dar.

Die erfindungsgemäßen Polyisocyanatgemische können z.B. durch Neutralisation eines Teils oder aller Carboxylgruppen mit beispielsweise tert. Aminen in eine wasserdispergierbare oder -lösliche Form überführt werden.

Für diese Neutralisationsreaktion geeignete tert. Amine sind beispielsweise gegenüber Isocyanatgruppen inerte tert. Amine wie Triethylamin, N-Methylpyrrolidin, N-Methylpiperidin oder N-Methylmorpholin oder gegenüber Isocyanatgruppen reaktionsfähige tert. Amine, insbesondere Aminoalkohole wie beispielsweise Triethanolamin, N-Methyl-diethanolamin, 2-(N,N-Dimethylamino)-iso-propanol oder N,N-Dimethylethanolamin.

Durch zumindest teilweise Neutralisation der Carboxylgruppen und anschließendes Lösen oder Dispergieren der erfindungsgemäßen Polyisocyanatgemische in Wasser resultieren wäßrige Lösungen oder Dispersionen von selbstvernetzenden Bindemitteln, die unter dem Einfluß des Wassers im Sinne eines Kettenverlängerungsmittels zu filmbildenden Lösungen oder Dispersionen hochmolekularer Kunststoffe ausreagieren (vgl. Beispiel 1). Im übrigen stellen die gegebenenfalls zumindest teilweise neutralisierten erfindungsgemäßen Polyisocyanatgemische unter dem Einfluß von Feuchtigkeit oder Hitze selbstvernetzende Bindemittel dar, die bei Raumtemperatur praktisch unbegrenzt lagerfähig sind.

Die erfindungsgemäßen Polyisocyanatgemische können auch mit den aus der Polyurethanchemie an sich bekannten höhermolekularen Polyhydroxylverbindungen zu hochmolekularen Polyurethanen umgesetzt werden, in denen neben freien Isocyanatgruppen die Uretdiongruppen bzw. Carboxylgruppen als weitere reaktive Zentren vorliegen, so daß die Polyurethane weiteren Reaktionen, insbesondere Vernetzungsreaktionen mit geeigneten Reaktionspartnern zugänglich sind.

Die erfindungsgemäßen Polyisocyanatgemische können auch als reaktive Emulgatoren wäßrigen Polymerdispersionen zwecks Verbesserung ihres Eigenschaftsniveaus zugesetzt werden.

Im Falle der zumindest teilweisen Überführung der Carboxylgruppen in Carboxylatgruppen, insbesondere durch eine Teilneutralisation mit tert. Aminen, entstehen erfindungsgemäße Polyisocyanatgemische, die auf Grund des Vorliegens der so eingeführten basischen Zentren bei ihrer erfindungsgemäßen Verwendung oftmals eine erwünschte katalytische Wirkung auf die Isocyanat-Additionsreaktion ausüben.

Bei der Verwendung der erfindungsgemäßen Polyisocyanatgemische insbesondere als Lackbindemittel oder als Zusatzmittel für Lacke können selbstverständlich die aus der Lacktechnologie üblichen Hilfs- und Zusatzmittel mitverwendet werden. Hierzu gehören beispielsweise Pigmente, Füllstoffe, Lösungsmittel, Verlaufhilfsmittel, Weichmacher u.dgl.

In den nachfolgenden Beispielen beziehen sich alle Prozentangaben, soweit nichts anderslautendes angemerkt, auf Gewichtsprozente.

Beispiel 1

1010,67 g eines Polyisocyanatgemischs, bestehend aus einem Gemisch aus Uretdiondiisocyanat und Isocyanuratgruppen aufweisendem Polyisocyanat jeweils auf Basis von 1,6-Diisocyanatohexan (HDI) mit einem NCO-Gehalt von 21,6 %, einer mittleren NCO-Funktionalität von 2,3 und einer Viskosität von 150 mPa.s/23˚ C.

46,90 g Dimethylolpropionsäure (DMPS) und
30,6 g N-Methylmorpholin (NMM)
werden unter Rühren vermischt und solange bei 70˚ C gehalten, bis der titrimetrisch bestimmte NCO-Gehalt des Reaktionsgemischs auf 15,3 % abgefallen ist.

Nach Abkühlen auf Raumtemperatur liegt ein erfindungsgemäßes Polyisocyanatgemisch teilweise in der Salzform vor.

```
Charakteristische Daten:
Viskosität (mPa.s/23° C):              1450
Farbzahl (Hazen):                        60
Uretdiongruppen (%):                   17,3
Carboxylgruppen (%):                    1,2
Carboxylatgruppen (%):                  0,2
```

Die auf eine Glasplatte gegebene Lösung des erfindungsgemäßen, im teilweise in der Salzform vorliegenden Polyisocyanatgemischs trocknet nach 2 Stunden bei Raumtemperatur oder 30 Minuten bei 140°C zu einem klaren, elastischen, riß- und klebfreien Film. Die Pendelhärten nach König betragen für den bei Raumtemperatur getrockneten Film 40 s, für den bei Hitze gehärteten Film 70 s.

Ein zum Vergleich auf eine Glasplatte gezogener Film des Ausgangspolyisocyanatgemischs bleibt unter den gleichen Trocknungsbedingungen flüssig.

Die Überprüfung der Lagerstabilität des erfindungsgemäßen, teilweise neutralisierten Polyisocyanatgemisches erfolgte durch Titration des NCO-Gehalts und Bestimmung der Viskosität in Abhängigkeit von der Lagerzeit bei Raumtemperatur:

| Lagerzeit (Tage) | NCO-Gehalt (%) | Viskosität (mPa.s, 23° C) |
|---|---|---|
| 0 | 15,3 | 1450 |
| 1 | 15,0 | 1460 |
| 5 | 14,9 | 1455 |
| 10 | 15,1 | 1450 |
| 30 | 14,8 | 1450 |
| 100 | 14,8 | 1465 |
| 150 | 14,6 | 1475 |

Zur Herstellung einer wäßrigen Dispersion werden 450 g des teilneutralisierten erfindungsgemäßen Polyisocyanatgemischs unter starkem Rühren mit 550 g entionisiertem Wasser versetzt. Es entsteht eine bläuliche, wäßrige Dispersion, die bis zum Ende der Gasentwicklung noch 1 h bei Raumtemperatur nachgerührt wird.

```
Charakteristische Daten:
Feststoffgehalt:                         44 %
mittlere Teilchengröße der
dispergierten Festkörper:               205 nm
Viskosität (DIN 4-Becher):               20 s
```

Die bei Raumtemperatur (a) und bei 140°C (b) getrockneten Filme der wäßrigen Polyurethan-/harnstoffdispersion sind klar, rißfrei und elastisch. Die Pendelhärten nach König liegen bei 50 (a) bzw. 80 s (b).

100 g des teilneutralisierten Polyisocyanatgemisches werden in einem 2 l Erlenmeyerkolben vorgelegt, mit 30 g entionisiertem Wasser versetzt, kurz umgerührt und stehengelassen. Unter $CO_2$-Entwicklung entsteht innerhalb 1 min ein aufsteigender poröser Schaumpilz.

Beispiel 2

1010,67 g des Ausgangspolyisocyanatgemischs gemäß Beispiel 1, 167,5 g DMPS und 130 g N-Methylpyrrolidon (NMP) werden vermischt und bei 80°C solange gerührt, bis der titrimetrisch bestimmte NCO-Wert auf 8,6 % abgesunken ist. Nach Abkühlen auf Raumtemperatur liegt eine klare Lösung eines erfindungsgemäßen Polyisocyanatgemischs in NMP vor.

**Charakteristische Daten:**

| | |
|---|---|
| Feststoffgehalt (%): | 90 |
| Carboxylgruppen (%): | 4,8 |
| Uretdiongruppen (%): | 16,0 |
| Viskosität (mPa.s/23°C): | 960 |

Das Polyisocyanatgemisch trocknet auf Glasplatten zu klaren, harten Filmen. Die Pendelhärten nach König liegen nach Raumtemperaturtrocknung bei 65 s, nach Ofentrocknung bei 85 s.

Die Lagerstabilitätsprüfung des erfindungsgemäßen Polyisocyanatgemischs bei Raumtemperatur ergab nach 150 Tagen einen NCO-Gehalt von 8,30 % und eine Viskosität von 975 mPa.s.

1308,2 g der Lösung des erfindungsgemäßen Polyisocyanatgemischs in NMP werden mit 40,1 g Dimethylethanolamin versetzt und während 5 Minuten verrührt.

Nach Zugabe von 1850 g entionisiertem Wasser entsteht eine milchig blaue, schwach durchsichtige wäßrige Dispersion mit einem Feststoffgehalt von 39,4 %, einer Viskosität (DIN 4-Becher) von 25 s bei einer mittleren Teilchengröße der dispergierten Partikel von 112 nm.

Beispiel 3 (Verwendung)

33 g eines hydroxyfunktionellen Polyacrylatharzes mit einem Hydroxylgruppengehalt von 4 % aus
30,6 Gew.-Teilen Hydroxyethylmethacrylat
17,4 Gew.-Teilen Methylmethacrylat
40,0 Gew.-Teilen Butylacrylat
8,0 Gew.-Teilen Acrylsäure
werden mit Ammoniaklösung und Wasser auf einen pH-Wert von 7,1 eingestellt, so daß eine 30 %ige Lösung des Polyacrylats in Wasser resultiert.

Nach Zugabe von 0,3 g eines handelsüblichen Verdickers (®Acrysol RM 8 der Firma Rohm und Haas, Frankfurt) und 0,7 g eines handelsüblichen Entschäumers (®Foamex 1498 der Firma Goldschmidt AG, Essen) zu der wäßrigen Acrylatlösung werden 43,4 g der mit 3 g Dimethylethanolamin neutralisierten Lösung des Polyisocyanatgemisches gemäß Beispiel 2 zugegeben. Die Mischung ist durch bloßes Einrühren leicht zu homogenisieren.

Ein auf eine Glasplatte aufgetragener Film des Gemisches trocknet nach ca. 2 h bei Raumtemperatur bis zur Klebfreiheit und bildet einen klaren, glänzenden und störungsfreien Lackfilm, der nach vollständiger Aushärtung eine Pendelhärte nach König von 130 s aufweist. Nach einminütigem Einwirken von aliphatischen Kohlenwasserstoffen bleibt die Filmoberfläche unverändert. Bei ebenso langer Einwirkung von Xylol, Methoxypropylacetat und Ethanol erfolgt eine geringfügige Anquellung der Lackoberfläche, wobei nach Verdunsten der Lösungsmittel eine Regeneration des Films erfolgt.

Ein entsprechend, jedoch ohne Mitverwendung der erfindungsgemäßen Polyisocyanatlösung hergestellter Film quillt bereits bei Einwirken von aliphatischen Kohlenwasserstoffen deutlich an und ist in aromatischen Kohlenwasserstoffen oder Methoxypropylacetat leicht löslich.

Die Verarbeitungszeit des erfindungsgemäßen, die erfindungsgemäße Polyisocyanatlösung enthaltenden Zweikomponenten-Systems liegt bei ca. 4 h.

Beispiel 4

7

100 g der in Beispiel 3 beschriebenen wäßrigen Polyacrylatlösung, der ebenso wie in Beispiel 3 Verdicker und Entschäumer zugegeben worden sind, werden mit 30,4 g eines Polyisocyanatgemisches homogenisiert. Bei dem Polyisocyanatgemisch handelt es sich um ein Gemisch aus 15,2 g eines Isocyanuratgruppen aufweisenden Polyisocyanats auf Basis von 1,6-Diisocyanatohexan mit einer NCO-Funktionalität von 3,5, einem NCO-Gehalt von 21,7 % und einer Viskosität bei 23° C von 1.500 mPa.s und 15,2 g des Polyisocyanatgemisches gemäß Beispiel 2, welches vorab mit 1,3 g N,N-Dimethylethanolamin neutralisiert worden ist.

Es liegt ein NCO/OH-Äquivalentverhältnis von 1,5:1 vor. Ein aus der Formulierung gewonnener Lackfilm ist nach 2 h bei Raumtemperatur klebfrei, hochglänzend und störungsfrei. Bei alleiniger Aushärtung des Polyacrylatharzes mit dem genannten, Isocyanuratgruppen aufweisenden Polyisocyanat ergeben sich Filme mit deutlicher Trübung und vermindertem Glanz.

Der erfindungsgemäße Lackfilm weist nach Aushärten bei Raumtemperatur eine Härte nach König von 150 s auf. Bei einminütigem Einwirken von aliphatischen oder aromatischen Kohlenwasserstoffen oder von Methoxypropylacetat zeigt sich keine Veränderung der Filmoberfläche. Bei Einwirkung von Ethanol während des gleichen Zeitraumes erfolgt eine geringfügige, nach Verdunsten des Lösungsmittels reversible Anquellung.

Beispiel 5 (katalysierte Oligomerisierung):

1500 g Hexamethylendiisocyanat (HDI) werden mit 60 g Dimethylpropionsäure (DMPS) vermischt und auf 55° C erwärmt. Nach Zugabe von 4,5 g Tri-n-butylphosphin und 4 h Rühren bei 60° C ist der NCO-Gehalt der klaren Lösung auf 37,5 % abgesunken.

Die Reaktion wird dann durch Zugabe von 4,1 g p-Toluolsulfonsäuremethylester (TSE) und einstündiges Nachrühren bei 80° C gestoppt.

Das überschüssige HDI wird anschließend durch Vakuumdestillation über einen Kurzwegverdampfer bei 150° C/0,05 mbar aus der Rohwarelösung entfernt.

Das in quantitativer Ausbeute erhaltene Carboxylpolyisocyanatgemisch hat folgende charakteristische Daten:

| | | | |
|---|---|---|---|
| NCO-Gehalt: | 19,50 % | Uretdiongruppen: | 14,5 % |
| Viskosität (23° C): | 2030 mPas | Carboxylgruppen: | 3,0 % |
| Farbzahl (Hazen): | 70 | | |
| Monomerengehalt: | 0,15 % fr. HDI | | |

Die Überprüfung der Lagerstabilität des erfindungsgemäßen, eingebaute Carboxylgruppen tragenden Polyisocyanatgruppengemisches ergab nach 150 Tagen bei Raumtemperatur einen NCO-Gehalt von 18,8 % und eine Viskosität von 2110 mPas (23° C).

Auf einer Glasplatte trocknet das erfindungsgemäße 100 %ige Polyisocyanatgemisch nach 1 h bei Raumtemperatur oder 30 Min. bei 140° C zu einem klaren, rißfreien und staubtrockenen Film.

Die Pendelhärte nach König beträgt für den bei Raumtemperatur getrockneten Film 60 s, für den hitzegehärteten 85 s.

200 g des beschriebenen Polyisocyanatgemisches werden mit 10 g N-Dimethylethanolamin versetzt und in 190 g entionisiertem Wasser dispergiert. Es entsteht eine schwach blaue wäßrige Polyurethan-Harnstoffdispersion, die bis zum Ende der Gasentwicklung nachgerührt wird und folgede charakteristische Daten aufweist:

| | | | |
|---|---|---|---|
| Feststoffgehalt: | 46,2 % | Pendelhärte (n. König): | |
| Viskosität (DIN-4-Becher): | 18 s | Raumtemp.Trocknung: | 80 s |
| mittl. Teilchengröße: | 195 nm | Ofentrocknung: | 95 s |

Beispiel 6 (katalysierte Oligomerisierung):

1100 g HDI und 373 g Isophorondiisocyanat (IPDI) werden mit 60 g DMPS vermischt und auf 55°C erwärmt.

Nach Zugabe von 4,5 g Tri-n-butylphosphin und 5 h Rühren bei 60°C ist der NCO-Gehalt der klaren Lösung auf 35,0 % gesunken.

Die Reaktion wird dann durch Zugabe von 4,1 g TSE und einstündiges Nachrühren bei 80°C abgestoppt.

Das überschüssige monomere Diisocyanatgemisch wird, wie in Beispiel 5 beschrieben, destilliert.

Das erhaltene Carboxylpolyisocyanatgemisch weist folgende charakteristische Daten auf:

```
NCO-Gehalt:           18,9 %     Uretdiongruppen: 13,3 %

Viskosität (23°C): 3180 mPas     Carboxylgruppen:  3,0 %

Farbzahl (Hazen):     75

Monomerengehalt:      0,10 % fr. HDI

                      0,12 % fr. IPDI
```

Die Überprüfung der Lagerstabilität des erfindungsgemäßen Carboxylpolyisocyanats ergab nach 150 Tagen bei Raumtemperatur einen NCO-Gehalt von 18,2 % und eine Viskosität von 3295 mPas.

Auf einer Glasplatte trocknet das erfindungsgemäße Carboxylpolyisocyanat nach 1 h bei Raumtemperatur oder 30 Min. bei 140°C zu einem klaren rißfreien Film.

Die Pendelhärten nach König betragen nach Raumtemperaturtrocknung 80 s, für den ofengetrockneten 98 s.

Beispiel 7

505,4 g des in Beispiel 1 beschriebenen Ausgangspolyisocyanats auf der Basis von HDI werden mit 579,0 g eines Uretdion-/Isocyanuratpolyisocyanats auf der Basis von 1-Isocyanato-3,3,5-trimethyl-5-isocyanato-methylcyclohexan (IPDI) abgemischt.

Die entstandene klare Lösung hat einen NCO-Gehalt von 20,2 %, eine Viskosität von 1320 mPas (23°C) und eine mittlere Funktionalität von 2.3.

```
Nach Zugabe von     46,80 g DMPS

                    30,60 g NMM     (vgl. Beispiel 1)
```

wird bei 74°C solange gerührt, bis der NCO-Gehalt des Reaktionsgemisches auf 14,9 % abgesunken ist.

Charakteristische Daten des erfindungsgemäßen Polyisocyanatgemisches :

```
Viskosität (mPas 123°C):   1795

Farbzahl (Hazen):            70

Uretdiongruppen:           11 %

Carboxylgruppen:           0,2 %

Carboxylatgruppen:         1,2 %
```

Die wie in Beispiel 1 hergestellten und getrockneten Filme des erfindungsgemäßen Polyisocyanatgemisches sind klar und homogen.

Die Pendelhärten nach König liegen nach 2 h Raumtemperaturtrocknung bei 85 s und nach 30 min Ofentrocknung bei 108 s. Nach einminütigem Einwirken von aliphatischen und aromatischen Kohlenwasserstoffen und Methoxypropylacetat bleibt die Filmoberfläche des ausgehärteten Polyisocyanatgemisches unverändert.

Die Überprüfung der Lagerstabilität des Polyisocyanatgemisches ergab nach 150 Tagen bei Raumtemperatur einen NCO-Gehalt von 14,6 % und eine Viskosität von 1811 mPas (23° C).

Nach Zugabe von Wasser im Überschuß lassen sich die erfindungsgemäßen Polyisocyanate wie in Beispiel 1 beschrieben, spontan dispergieren.

**Patentansprüche**

1. Polyisocyanatgemische, gekennzeichnet durch
   a) eine mittlere NCO-Funktionalität von 1,5 bis 4,0,
   b) einen Gehalt an (cyclo)aliphatisch gebundenen Isocyanatgruppen (NCO, Molekulargewicht = 42) von 2 bis 40 Gew.-%,
   c) einen Gehalt an chemisch fixierten Carboxylgruppen (COOH, Molekulargewicht = 45) von 0,01 bis 15 Gew.-% und
   d) einen Gehalt an (cyclo)aliphatisch gebundenen Uretdiongruppen ($C_2N_2O_2$, Molekulargewicht = 84) von 1 bis 23 Gew.-%.

2. Verfahren zur Herstellung von Polyisocyanatgemischen gemäß Anspruch 1 durch Umsetzung einer Polyisocyanatkomponente A), bestehend aus mindestens einem Polyisocyanat mit (cyclo)aliphatisch gebundenen Isocyanatgruppen des Molekulargewichtsbereichs 168 bis 1000 mit einer HydroxycarbonsäureKomponente B), bestehend aus mindestens einer aliphatischen Hydroxycarbonsäure des Molekulargewichtsbereichs 76 bis 200, gegebenenfalls unter Mitverwendung von weiteren Reaktionspartnern mit gegenüber Isocyanatgruppen reaktionsfähigen, aliphatisch gebundenen Hydroxylgruppen unter Einhaltung eines Äquivalentverhältnisses von Isocyanatgruppen zu Hydroxylgruppen von 1,05:1 bis 80:1 und gegebenenfalls destillative Entfernung von gegebenenfalls im Überschuß verwendeten Ausgangsdiisocyanaten und gegebenenfalls anschließende Abmischung des resultierenden Umsetzungsprodukts mit weiteren organischen Polyisocyanaten C), dadurch gekennzeichnet, daß man als Polyisocyanatkomponente A) oder als Teil der Polyisocyanatkomponente A) und/oder als Polyisocyanate C) oder als Teil der Polyisocyanate C) (cyclo)aliphatisch gebundene Uretdiongruppen aufweisende Diisocyanate mit (cyclo)aliphatisch gebundenen Isocyanatgruppen verwendet, wobei im übrigen Art und Mengenverhältnisse der Reaktionspartner so gewählt werden, daß die resultierenden Polyisocyanatgemische den in Anspruch 1 unter a) bis d) genannten Bedingungen entsprechen.

3. Verfahren zur Herstellung von Polyisocyanatgemischen gemäß Anspruch 1, dadurch gekennzeichnet, daß man eine Polyisocyanatkomponente A), bestehend aus mindestens einem, im wesentlichen Uretdiongruppen-freien Polyisocyanat mit (cyclo)aliphatisch gebundenen Isocyanatgruppen des Molekulargewichtsbereichs 168 bis 1000 mit einer Hydroxycarbonsäure-Komponente B), bestehend aus mindestens einer aliphatisch gebundenen Hydroxycarbonsäure des Molekulargewichtsbereichs 76 bis 200, gegebenenfalls unter Mitverwendung von weiteren Reaktionspartnern mit gegenüber Isocyanatgruppen reaktionsfähigen, aliphatisch gebundenen Hydroxylgruppen unter Einhaltung eines Äquivalentverhältnisses von Isocyanatgruppen zu Hydroxylgruppen von 1,05:1 bis 400:1 umsetzt, die verbleibenden Isocyanatgruppen des Umsetzungsprodukts zu 5 bis 60 % in Gegenwart eines die Dimerisierung von Isocyanatgruppen beschleunigenden Katalysators in Uretdiongruppen und gegebenenfalls Isocyanuratgruppen überführt, das dann vorliegende Reaktionsgemisch gegebenenfalls von überschüssigem Ausgangspolyisocyanate A) befreit und/oder mit weiteren organischen Polyisocyanaten C) abmischt, wobei Art und Mengenverhältnisse der Reaktionspartner und die Menge des gegebenenfalls am Ende der Umsetzung entfernten Ausgangspolyisocyanats A) und/oder des dem Reaktionsgemisch zugesetzten Polyisocyanat C) so bemessen werden, daß die resultierenden Polyisocyanatgemische den oben unter a) bis d) genannten Bedingungen entsprechen.

4. Verwendung der Polyisocyanatgemische gemäß Anspruch 1, gegebenenfalls in zumindest teilweise mit tertiären Aminen neutralisierter Form als Bindemittel für unter dem Einfluß von Feuchtigkeit und/oder Hitze vernetzbare Überzugsmitteln.

5. Verwendung der Polyisocyanatgemische gemäß Anspruch 1, gegebenenfalls in zumindest teilweise mit tertiären Aminen neutralisierter Form, als Reaktionspartner für Verbindungen mit gegenüber Isocyanatgruppen und/oder Carboxylgruppen reaktionsfähigen Gruppen bei der Herstellung von hochmolekularen Kunststoffen.

**Europäisches**
**Patentamt**

**EUROPÄISCHER**
**RECHERCHENBERICHT**

Nummer der Anmeldung

**EP 90 12 4222**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | EP-A-0 277 483 (BAYER) <br> * Ansprüche 1-4; Seite 2, Zeile 42 - Seite 3, Zeile 23 * <br> – – – | 1,2,4,5 | C 08 G 18/79 <br> C 08 G 18/80 <br> C 07 D 229/00 <br> C 08 G 18/08 <br> C 09 D 175/04 |
| A | GB-A-1 153 815 (BAYER) <br> * Anspruch 6; Seite 1, Zeile 15 - Seite 2, Zeile 7; Seite 2, Zeilen 27-31 * <br> – – – | 1,3 | |
| A | FR-A-2 253 039 (BAYER) <br> * Ansprüche 1,9; Seite 5, Zeilen 6-21 * <br> – – – | 1 | |
| A | DE-A-2 538 484 (VEBA-CHEMIE) <br> * Anspruch; Seite 3, letzter Absatz - Seite 4, Absatz 1 * <br> – – – | 1 | |
| A | US-A-4 496 684 (J.M. O'CONNOR et al.) <br> * Anspruch 1; Spalte 1, Zeile 56 - Spalte 2, Zeile 25 * <br> – – – | 1 | |
| D,A | US-A-3 412 054 (C.L. MILLIGAN et al.) <br> * Anspruch 1; Spalte 3, Zeilen 24-33; Beispiel 1 * <br> – – – – – | 1 | |

|  | RECHERCHIERTE SACHGEBIETE (Int. Cl.5) |
|---|---|
|  | C 08 G <br> C 07 D |

Der vorliegende Recherchenbericht wurde für alle Patentansprüche erstellt

| Recherchenort | Abschlußdatum der Recherche | Prüfer |
|---|---|---|
| Den Haag | 02 Mai 91 | VAN PUYMBROECK M.A. |